# EUROPÄISCHE PATENTSCHRIFT

(11) **EP 2 305 105 B1**
(45) Veröffentlichungstag und Bekanntmachung des Hinweises auf die Patenterteilung: **16.05.2012**
(21) Anmeldenummer: 09012550.1
(22) Anmeldetag: 05.10.2009
(51) Int. Cl.: A61B 5/00

(54) **Verfahren zur Erkennung einer Fehlfunktion eines Sensors zur in-vivo Messung einer Analytkonzentration**
Methods for the detection of a malfunction in a sensor for in-vivo analyte measurement
Méthode de détection d'un dysfonctionnement d'un capteur permettant la mesure in-vivo de la concentration d'un analyte

(43) Veröffentlichungstag der Anmeldung: 06.04.2011
(73) Patentinhaber: Roche Diagnostics GmbH, 68305 Mannheim (DE); F.Hoffmann-La Roche AG, 4070 Basel (CH)
(72) Erfinder: Schmelzeisen-Redeker, Günther, 64653 Lorsch (DE); Staib, Arnulf, 64646 Heppenheim (DE); Kloetzer, Hans-Martin, 68199 Mannheim (DE)
(74) Vertreter: Twelmeier Mommer & Partner

(56) Entgegenhaltungen:
- US-A1- 2009 156 924

## Beschreibung

Die Erfindung betrifft ein Verfahren zur Erkennung einer Fehlfunktion eines Sensors zur in-vivo Messung einer Analytkonzentration, wobei mit dem Sensor eine Serie von Messsignalen erzeugt wird und aus den Messsignalen fortlaufend ein Wert eines Rauschparameters bestimmt wird, der angibt, wie stark die Messung durch Störsignale beeinträchtigt ist.

Ein solches Verfahren ist aus der US 2009/0076361 A1 bekannt. Bei dem bekannten Verfahren wird ein Rauschparameter mit einem vorgegebenen Schwellenwert verglichen. Übersteigt der Wert des Rauschparameters den Schwellenwert, wird auf eine Fehlfunktion geschlossen. Ein Verfahren mit den im Oberbegriff des Anspruchs 1 angegebenen Merkmalen ist auch aus der US 2009/156924 A1 bekannt

Ziel bei der Überwachung von Sensoren zur in-vivo Messung von Analytkonzentrationen ist es, eventuelle Fehlfunktionen möglichst frühzeitig und zuverlässig zu erkennen. Aufgabe der vorliegenden Erfindung ist es, einen Weg aufzuzeigen, wie dieses Ziel noch besser erreicht werden kann.

Diese Aufgabe wird durch ein Verfahren mit den im Anspruch 1 angegebenen Merkmalen gelöst. Vorteilhafte Weiterbildungen der Erfindung sind Gegenstand von Unteransprüchen.

Bei einem erfindungsgemäßen Verfahren wird aus fortlaufend ermittelten Werten des Rauschparameters ermittelt, wie schnell sich der Rauschparameter ändert und die Änderungsgeschwindigkeit des Rauschparameters zur Erkennung einer Fehlfunktion ausgewertet. Auf diese Weise kann eine Fehlfunktion wesentlich zuverlässiger als durch Vergleich des Rauschparameters mit einem fest vorgegebenen Schwellenwert ermittelt werden.

Mit implantierbaren Sensoren können Analytkonzentrationen im menschlichen Körper kontinuierlich oder quasi-kontinuierlich gemessen werden. Von besonderem Interesse sind dabei Analyten, die sich im Zeitraum von Stunden oder Tagen signifikant ändern, wie dies beispielsweise bei Glucose der Fall ist. Sensoren zur in-vivo Messung liefern eine Serie von Messsignalen, beispielsweise Strom- oder Spannungswerte, die über einen funktionalen Zusammenhang mit dem zu bestimmenden Wert der gesuchten Analytkonzentration verbunden sind und diesen nach einer Kalibration widerspiegeln.

Wie bei jeder Messung sind auch die konzentrationsabhängigen Messsignale von in-vivo Sensoren durch Messfehler beeinträchtigt. Neben systematischen Messfehlern, die oft zu einer konsistenten Abweichung führen, sind insbesondere zufällige Messfehler von Bedeutung, die unter dem Begriff Rauschen zusammengefasst werden. Als Rauschen werden dabei sowohl Messfehler bezeichnet, die ihren Ursprung in dem Sensor selbst haben, z.B. elektronischen Rauschen, als auch Messfehler, die auf einer unkontrollierten Einwirkung auf den Sensor, beispielsweise durch Bewegungen, oder einer vorübergehenden Abweichung der Analytkonzentration in der Umgebung des Sensors von der Analytkonzentration an anderen Stellen im Körper des Patienten beruhen.

Wie stark eine Messung durch Rauschen beeinträchtigt ist, lässt sich durch einen Rauschparameter quantifizieren, der beispielsweise als Standardabweichung eines Störsignalanteils berechnet werden kann. Zur Berechnung des Rauschparameters wird deshalb in der Regel zunächst ermittelt, welcher Anteil eines Messwertes auf Störsignalen beruht. Im einfachsten Fall kann näherungsweise davon ausgegangen werden, dass ein gegebener Messwert die Summe eines Nutzsignals, das der gesuchten Analytkonzentration entspricht, und eines Störsignals ist. Zur Trennung des Rauschanteils vom Nutzanteil können beispielsweise rekursive Filter, insbesondere Kalmanfilter, oder Savitzky-Golay-Filter verwendet werden.

Durch Differenzbildung zwischen dem Messwert und einem ermittelten Wert des Nutzanteils zur Zeit t wird der Rauschanteil erhalten. Das so ermittelte Rauschen enthält umso weniger Nutzsignal-Anteile, je präziser der Nutzanteil ermittelt wurde.

Hat man aus einer Serie von Werten den Rauschanteil erhalten, kann man eine Serie von Werten eines Rauschparameters berechnen, der das Rauschen quantifiziert. Der Rauschparameter kann beispielsweise als Standardabweichung der Rauschsignalwerte in einem vorgegebenen Intervall berechnet werden. Statt der Standardabweichung können als Rauschparameter beispielsweise auch Varianzen, Variationskoeffizienten, Interquartil-Bereiche oder ähnliches verwendet werden.

Aus den fortlaufend ermittelten Werten des Rauschparameters lässt sich ermitteln, wie schnell sich der Rauschparameter ändert und die Änderungsgeschwindigkeit des Rauschparameters zur Erkennung einer Fehlfunktion auswerten. Als Folge des Erkennens einer Fehlfunktion kann beispielsweise ein Warnsignal erzeugt werden. Mit einem solchen Warnsignal kann ein Benutzer auf das Vorliegen einer Fehlfunktion aufmerksam gemacht werden. Alternativ oder zusätzlich kann das Warnsignal auch bewirken, dass das Messsystem keine Messwerte mehr anzeigt oder ermittelte Messwerte in einem Speicher des Systems als unzuverlässig markiert werden.

Im Allgemeinen werden Änderungsgeschwindigkeiten als zeitliche Ableitung der sich ändernden Größe bestimmt. Numerisch lässt sich eine zeitliche Ableitung am einfachsten dadurch ermitteln, dass die Differenz zwischen zwei aufeinanderfolgenden Größen berechnet und durch den zeitlichen Abstand, in dem die beiden Werte aufeinanderfolgen, dividiert wird. Zur Bestimmung der Änderungsgeschwindigkeit eines Rauschparameters ist diese Vorgehensweise allerdings eher weniger geeignet. Dies liegt daran, dass der Rauschparameter selbst einem starken Rauschen unterliegt und sich deshalb zwischen unmittelbar aufeinanderfolgenden Rauschparameterwerten relativ große Unterschiede ergeben können, denen keine signifikante Änderung des Sensors oder der Sensorumgebung entspricht. Bevorzugt wird die Änderungsgeschwindigkeit des Rauschparameters deshalb an einer geglätteten Serie von Werten des Rauschparameters bestimmt.

Eine Glättung kann beispielsweise durch Mittelwertbildung einer vorgegebenen Anzahl von aufeinander folgenden Werten des Rauschparameters vorgenommen werden. Möglich ist es auch, eine Serie von Rauschparameterwerten mit einem rekursiven Filter, beispielsweise einem Kalmanfilter, zu glätten. An einer geglätteten Serie von Rauschparameterwerten kann ein Maß für die Änderung der Geschwindigkeit des Rauschparameters dann beispielsweise durch Differenzbildung aufeinanderfolgender Werte berechnet werden. Rekursive Filter, insbesondere Kalmanfilter, können auch zur Glättung einer Serie von Werten der Änderungsgeschwindigkeit verwendet werden, damit diese leichter ausgewertet werden können.

Die Änderungsgeschwindigkeit des Rauschparameters kann mittels einer Bewertungsfunktion ausgewertet werden. Ein einfaches Beispiel einer Bewertungsfunktion ist eine Stufenfunktion. Mit einer Stufenfunktion kann ein Schwellenwert vorgegeben werden, mit dem die Änderungsgeschwindigkeit des Rauschparameters verglichen wird. Indem der Schwellenwert geeignet gewählt wird, kann aus einem Überschreiten des Schwellenwertes auf eine Fehlfunktion geschlossen werden. Es können auch kontinuierliche Bewertungsfunktionen verwendet, die beispielsweise den momentanen Grad Zuverlässigkeit von Messwerten angeben. Hierfür kann mit der Bewertungsfunktion eine Abbildung, insbesondere eine nicht-lineare Abbildung, auf ein vorgegebenes Intervall, beispielsweise von 0 bis 1 oder von 0 bis 100 vorgenommen werden.

Eine vorteilhafte Weiterbildung der Erfindung sieht vor, dass der Schwellenwert während des Betriebs des Sensors in Abhängigkeit von einem Messergebnis geändert wird. Dieses Messergebnis kann aus Messsignalen des Sensors ermittelt werden, also beispielsweise die Analytkonzentration oder den Wert des Rauschparameters angeben. Bevorzugt wird dabei durch Auswerten wenigstens eines drauffolgenden Wertes der Änderungsgeschwindigkeit oder eines darauffolgenden Wertes des Rauschparameters überprüft, ob die Überschreitung signifikant ist.

Bei einem elektrochemischen Sensor, der eine Arbeitselektrode, eine Gegenelektrode und eine Referenzelektrode aufweist, kann das Messergebnis, in Abhängigkeit von welchem der Schwellenwert geändert wird, vorteilhaft auf einer Messung des elektrischen Potentials der Gegenelektrode beruhen. Durch eine Messung des elektrischen Potentials der Gegenelektrode kann beispielsweise die elektrische Spannung zwischen der Arbeitselektrode und der Gegenelektrode oder zwischen der Gegenelektrode und der Referenzelektrode ermittelt werden. Wie in der EP 2 030 561 A1 beschrieben wird, kann durch eine Messung des elektrischen Potentials der Gegenelektrode eine Fehlfunktion eines Sensors erkannt werden. Indem bei der Auswertung eines Rauschparameters auch das Potential der Gegenelektrode berücksichtigt wird, kann deshalb eine Fehlfunktion zuverlässiger und schneller erkannt werden. Beispielsweise kann der Schwellenwert, mit dem die Änderungsgeschwindigkeit des Rauschparameters verglichen wird, herabgesetzt werden, sobald eine Messung des elektrischen Potentials der Gegenelektrode verdächtige Werte ergibt, die eine Fehlfunktion plausibel erscheinen lassen, aber noch nicht zuverlässig erkennen lassen.

Bevorzugt wird eine durch Auswertung der Anderungsgeschwindigkeit ermittelte Fehlfunktion einer von zwei oder mehr Klassen zugeordnet. Beispielsweise kann als Konsequenz einer Zuordnung zu einer ersten Klasse ein erstes Warnsignal erzeugt werden, während als Konsequenz einer Zuordnung zu einer zweiten Klasse ein zweites Warnsignal erzeugt wird. Mit einem ersten Warnsignal kann beispielsweise eine weniger schwerwiegende Fehlfunktion, die möglicherweise von selbst wieder verschwindet, angezeigt werden, während mit dem zweiten Warnsignal eine schwerwiegendere Fehlfunktion signalisiert werden kann. Beispielsweise können für das erste und das zweite Warnsignal Signallichter unterschiedlicher Farben, beispielsweise gelb und rot und/oder akustische Signale unterschiedlicher Intensität verwendet werden. Das zweite Warnsignal kann beispielsweise auch eine Abschaltung einer Anzeige aktueller Messwerte der Analytkonzentration bewirken.

Die Zuordnung einer Fehlfunktion zu einer von mehreren Klassen kann im einfachsten Fall durch unterschiedliche Schwellenwerte erfolgen. Überschreitet die Änderungsgeschwindigkeit einen ersten Schwellenwert, erfolgt eine Zuordnung der Fehlfunktion zu der ersten Klasse. Ist die Änderungsgeschwindigkeit so groß, dass auch der zweite Schwellenwert überschritten wird, erfolgt eine Zuordnung der Fehlfunktion zu der zweiten Klasse.

Die Zuordnung einer Fehlfunktion zu einer zweiten Klasse kann auch von einem weiteren Parameter abhängen, der mit einem weiteren Schwellenwert verglichen wird. Der weitere Parameter kann beispielsweise eine Zeitdauer sein, während welcher die Änderungsgeschwindigkeit den Schwellenwert überschreitet. Die Zuordnung einer Fehlfunktion zu der zweiten Klasse kann also davon abhängig gemacht werden, wie lange die Änderungsgeschwindigkeit einen vorgegebenen Schwellenwert überschreitet. Der weitere Parameter kann beispielsweise auch der Rauschparameter selbst sein oder bei Verwendung eines elektrochemischen Sensors durch eine Messung des Potentials der Gegenelektrode ermittelt werden.

Der erfindungsgemäß verwendete Rauschparameter kann eine dimensionslose Größe sein und das Rauschen in Relation zur Intensität eines Nutzsignals angeben. Bei dieser Vorgehensweise entspricht der Rauschparameter dem in weiten Bereichen der Technik verwendeten Signalrauschverhältnis. Bei einem erfindungsgemäßen Verfahren charakterisiert der Rauschparameter jedoch bevorzugt die absolute Intensität der Störsignale. Dies bedeutet, dass bei der Berechnung des Rauschparameters der Störsignalanteil nicht mit dem Nutzsignal normiert wird. Ein Anstieg des Nutzsignals, also ein Anstieg der Analytkonzentration, führt dann nicht notwendigerweise zu einem kleineren Rauschparameter, sondern kann diesen unbeeinflusst lassen.

Ein weiterer Aspekt der vorliegenden Erfindung betrifft ein Verfahren zur Erkennung einer Fehlfunktion eines Sensors zur in-vivo Messung einer Analytkonzentration, wobei mit dem Sensor eine Serie von Messsignalen erzeugt wird, aus den Messsignalen fortlaufend ein Wert eines Rauschparameters bestimmt wird, der angibt, wie stark die Messsignale durch Störsignale beeinträchtigt sind, und der Rauschparameter zur Erkennung einer Fehlfunktion mit einem Schwellenwert verglichen wird, der während des Betriebs des Sensors in Abhängigkeit von einem Messergebnis geändert wird.

Dieses Verfahren kann mit dem vorstehend beschriebene Verfahren kombiniert werden, indem es mit Merkmalen des vorstehend beschriebenen Verfahrens ausgestaltet wird. Insbesondere kann der Schwellenwert während des Betriebs des Sensors in Abhängigkeit von einem Messergebnis geändert werden. Dieses Messergebnis kann aus Messsignalen des Sensors ermittelt werden, also beispielsweise die Analytkonzentration oder den Wert des Rauschparameters angeben, oder bei einem elektrochemischen Sensor auf einer Messung des elektrischen Potentials der Gegenelektrode beruhen.

Unabhängig davon, wie im Detail durch Auswertung eines Rauschparameters eine Fehlfunktion eines in-vivo Sensors ermittelt wird, ist im Allgemeinen ein möglichst geringes Rauschen vorteilhaft. Um das Rauschen zu reduzieren, kann aus mehreren Messsignalen der Analytkonzentration jeweils ein Messwert, beispielsweise durch Mittelung, berechnet und aus mehreren Messwerten jeweils ein Wert des Rauschparameters berechnet werden. Messsignale können mit einem in-vivo Sensor quasi kontinuierlich erzeugt werden. Vorteilhaft ist es insbesondere mehr als fünf Messsignale pro Minute zu erzeugen, beispielsweise mehr als 10 Messsignale. Durch Mittelung können aus den Messsignalen Messwerte berechnet werden, die wesentlich weniger durch Rauschen beeinträchtigt sind als die Messsignale. Die Messwerte können dabei für auf einander folgende Zeitintervalle berechnet werden, indem zur Berechnung eines Messwerts jeweils alle in dem betreffenden Zeitintervall gemessenen Messsignale berücksichtigt werden. Anstelle von aufeinander folgenden Zeitintervallen können aber auch gleitende, also überlappende, Zeitintervalle verwendet werden.

Weitere Einzelheiten und Vorteile der Erfindung werden an einem Ausführungsbeispiel der Erfindung unter Bezugnahme auf die beigefügten Zeichnungen erläutert. Es zeigen:
- Figur 1: ein Beispiel einer Serie von Messwerten der Glucosekonzentration,
- Figur 2: den Rauschanteil der in Figur 1 gezeigten Serie;
- Figur 3: den Verlauf des Rauschparameters zu der in Figur 1 gezeigten Serie,
- Figur 4: den Verlauf des Rauschparameters nach Glättung; und
- Figur 5: den Verlauf der Änderungsgeschwindigkeit des Rauschparameters.

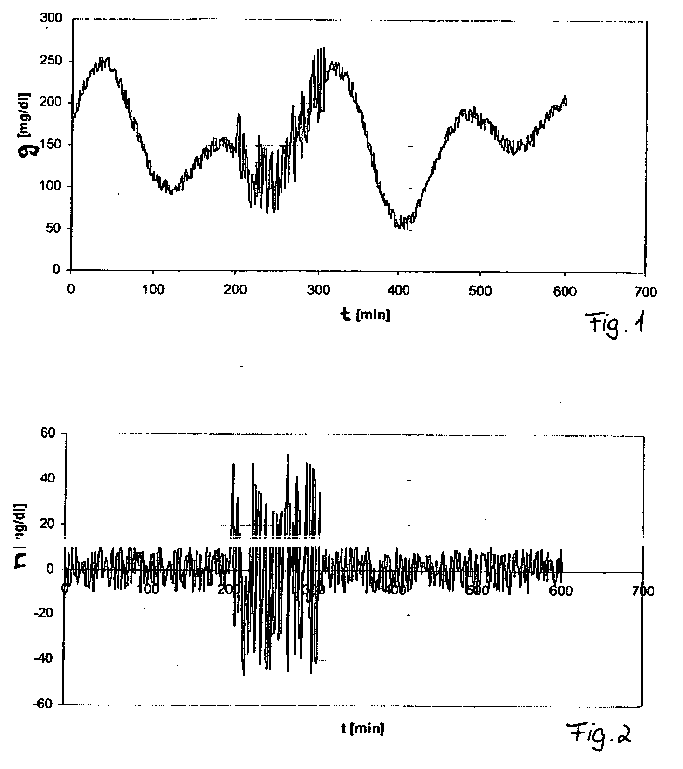

Figur 1 zeigt ein Beispiel einer Serie von Messwerten der Glucosekonzentration g über der Zeit t. Die Messwerte wurden mit einem elektrochemischen Sensor in-vivo ermittelt, wobei pro Minute etwa 30 bis 100 Messsignale erzeugt wurden, aus denen jeweils ein Messwert als arithmetisches Mittel berechnet wurde. Die dargestellten Messwerte wurden jeweils für aufeinander folgende Zeitintervalle von jeweils einer Minute berechnet.

Der in Figur 1 dargestellte Verlauf der Messwerte der Glucosekonzentration g ist durch Rauschen beeinträchtigt. Mittels eines rekursiven Filters, beispielsweise eines Kalman-Filters, wurde der Rauschanteil der in Figur 1 dargstellten Serie von Messwerten ermittelt. Der Rauschanteil n ist in Figur 2 in mg/dl über der Zeit t in Minuten dargestellt. Idealerweise ist der Rauschanteil dabei jeweils die Abweichung des Messwerts der Glucosekonzentration von der tatsächlichen bzw. vermuteten Glucosekonzentration g, welche durch Auswertung des Verlaufs der Messwerte ermittelt wurde, beispielsweise durch Anwendung eines Kalman-Filters.

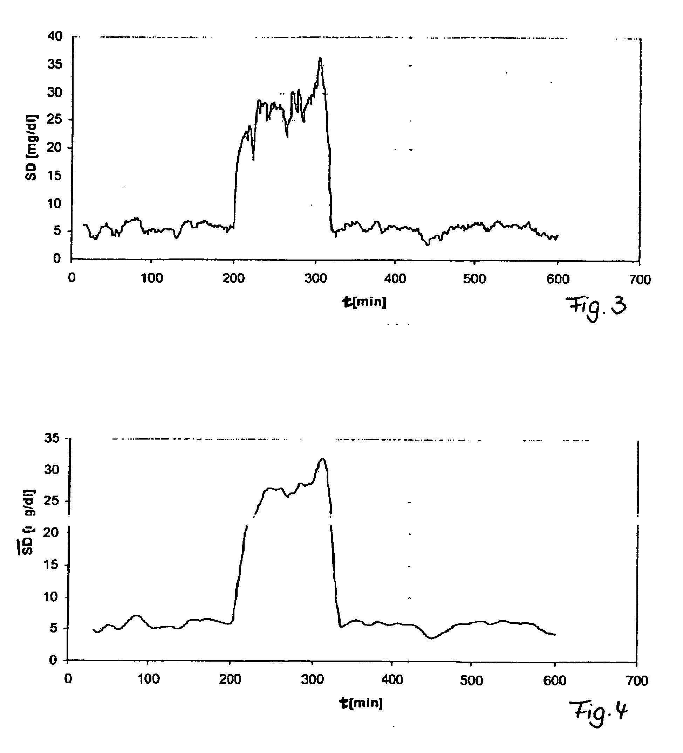

Aus dem in Figur 2 dargestellten Rauschanteil n kann ein Rauschparameter berechnet werden, der angibt, wie stark die Messung durch Störsignale beeinträchtigt ist. Als Rauschparameter kann insbesondere die Standardabweichung der für ein Zeitintervall ermittelten Rauschanteile verwendet werden. In Figur 3 ist über der Zeit t in Minuten die Standardabweichung SD als Rauschparameter zu dem in Figur 2 gezeigten Verlauf des Rauschanteils, dessen zeitlicher Mittelwert Null ist, dargestellt. Die Standardabweichung wurde bei dem dargestellten Beispiel für gleitende Zeitfenster von beispielsweise 15 Minuten berechnet. Allgemein wird der Rauschparameter bevorzugt für gleitende Zeitfenster von mindestens 5 Minuten berechnet, beispielsweise für Zeitfenster von 5 bis 30 Minuten, insbesondere 10 bis 20 Minuten.

Wie Figur 3 zeigt, ist der Rauschparameter SD selbst ebenfalls durch Rauschen beeinträchtigt. Vor einer weiteren Auswertung des Rauschparameters kann es deshalb vorteilhaft sein, die Serie von Rauschparameterwerten zu glätten. Dies kann beispielsweise durch Mittelung von Rauschparameterwerten über ein vorgegebenes Zeitfenster erfolgen. Der in Figur 3 dargestellte Verlauf der Rauschparameterwerte wurde geglättet, indem jeweils über alle Rauschparameterwerte in einem gleitenden Zeitfenster von beispielsweise 15 Minuten gemittelt wurden. Das Ergebnis dieser Glättung, nämlich die für die Zeitfenster berechneten Mittelwerte *S̅D̅*, ist in Figur 4 dargestellt. In der Regel ist es vorteilhaft, den Rauschparameter SD mit gleitenden Zeitfenstern von mindestens 5 Minuten zu glätten, beispielsweise mit Zeitfenstern von 5 bis 30 Minuten, insbesondere 10 bis 20 Minuten.

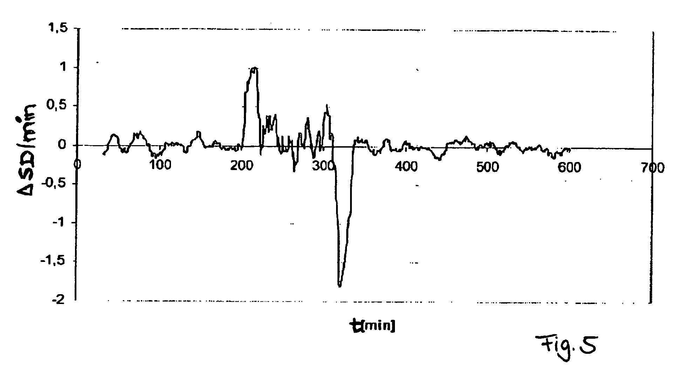

Aus dem Verlauf der Rauschparameterwerte SD bzw. der geglätteten Rauschparameterwerte lässt sich ermitteln, wie schnell sich der Rauschparameter n ändert. Figur 5 zeigt die so ermittelte Änderungsgeschwindigkeit des Rauschparameters SD. Die Änderungsgeschwindigkeit des Rauschparameters kann beispielsweise als Ableitung der Figur 4 dargestellten Verlaufskurve ermittelt werden. Numerisch lässt sich die Abteilung nach der Zeit als Differenz aufeinander folgender Werte berechnen, wobei die Differenz durch die Zeit zwischen den betreffenden Werten dividiert wird. Bei einer Serie von äquidistanten Werten ist die Änderungsgeschwindigkeit deshalb der Differenz zwischen aufeinander folgenden Werten proportional und in Figur 5 deshalb mit ΔSD bezeichnet.

Mit bloßem Auge lässt sich in Figur 1 und insbesondere in Figur 2 erkennen, dass zwischen der Zeit t von etwa 200 Minuten und etwa 300 Minuten ein stark erhöhtes Rauschen aufgetreten ist. Dieses erhöhte Rauschen zeigt sich besonders deutlich in den Figuren 3 und 4. Um den Beginn des erhöhten Rauschens präzise zu ermitteln, ist insbesondere die in Figur 5 dargestellte Änderungsgeschwindigkeit des Rauschparameters geeignet.

In Figur 5 zeigt sich eine Zunahme der Änderungsgeschwindigkeit ΔSD des Rauschens als eine Spitze, die sich deutlich von dem Untergrund abhebt. Das Ende des erhöhten Rauschens zeigt sich entsprechend als eine nach unten zeigende Spitze. Durch Auswertung der Änderungsgeschwindigkeit ΔSD kann folglich frühzeitig und zuverlässig ein erhöhtes Rauschen festgestellt werden und daraus auf eine Fehlfunktion des Sensors geschlossen werden. Beispielsweise kann dafür die Änderungsgeschwindigkeit des Rauschens mit einem vorgegebenen Schwellenwert verglichen werden. Übersteigt die Änderungsgeschwindigkeit des Rauschens einen vorgegebenen Schwellenwert von beispielsweise der halben Standardabweichung pro Minute, so wird ein Warnsignal erzeugt.

Der Auswertung der Änderungsgeschwindigkeit des Rauschens kann durch eine Auswertung der absoluten Rauschintensität, beispielsweise einer Schwelle für den Rauschparameter, oder der Auswertung einer Messung des elektrischen Potentials der Gegenelektrode ergänzt werden, insbesondere um die Schwere der Störung zu beurteilen.

Bei einer eher leichten Störung, wie sie in dem vorstehend beschriebenen Ausführungsbeispiel auftritt, kann ein einfaches Warnsignal eine angemessene Reaktion auf die erkannte Fehlfunktion des Sensors darstellen. Bei einer schwerwiegenderen Fehlfunktion, die sich durch intensiveres Rauschen auszeichnet, kann beispielsweise ein Alarmsignal erzeugt werden und/oder die während erhöhten Rauschens ermittelten Messwerte der Glucosekonzentration g als unzuverlässig verworfen werden.

## Patentansprüche

1. Verfahren zur Erkennung einer Fehlfunktion eines Sensors zur in-vivo Messung einer Analytkonzentration, wobei
mit dem Sensor eine Serie von Messsignalen erzeugt wird, und
aus den Messsignalen fortlaufend ein Wert eines Rauschparameters bestimmt wird, der angibt, wie stark die Messung durch Störsignale beeinträchtigt ist, **dadurch gekennzeichnet, dass** aus fortlaufend ermittelnden Werten des Rauschparameters ermittelt wird, wie schnell sich der Rauschparameter ändert und die Änderungsgeschwindigkeit des Rauschparameters zur Erkennung einer Fehlfunktion des Sensors ausgewertet wird.

2. Verfahren nach Anspruch 1, **dadurch gekennzeichnet, dass** jeweils für ein vorgegebenes Zeitintervall ein Wert des Rauschparameters ermittelt wird.

3. Verfahren nach einem der vorstehenden Ansprüche, **dadurch gekennzeichnet, dass** aus den Messsignalen mit einem rekursiven Filter fortlaufend ein Wert des Rauschparameters bestimmt wird.

4. Verfahren nach einem der vorstehenden Ansprüche, **dadurch gekennzeichnet, dass** die Änderungsgeschwindigkeit des Rauschparameters durch Vergleich mit einem Schwellenwert ausgewertet wird.

5. Verfahren nach Anspruch 4, **dadurch gekennzeichnet, dass** der Schwellenwert während des Betriebs des Sensors in Abhängigkeit von einem Messergebnis geändert wird.

6. Verfahren nach Anspruch 5, **dadurch gekennzeichnet, dass** der Sensor eine Arbeitselektrode, eine Gegenelektrode und eine Referenzelektrode aufweist, wobei das Messergebnis auf einer Messung des elektrischen Potentials der Gegenelektrode beruht.

7. Verfahren nach einem der Ansprüche 4 bis 6, **dadurch gekennzeichnet, dass** nach einer Feststellung, dass ein Wert der Änderungsgeschwindigkeit den Schwellenwert überschreitet, durch Auswerten wenigstens eines darauf folgenden Wertes der Änderungsgeschwindigkeit überprüft wird, ob die Überschreitung signifikant ist.

8. Verfahren nach einem der vorstehenden Ansprüche, **dadurch gekennzeichnet, dass** als Folge des Erkennens einer Fehlfunktion ein Warnsignal erzeugt wird.

9. Verfahren nach einem der vorstehenden Ansprüche, **dadurch gekennzeichnet, dass** eine durch Auswertung der Änderungsgeschwindigkeit ermittelte Fehlfunktion einer von mindestens zwei Klassen zugeordnet wird.

10. Verfahren nach Anspruch 9, **dadurch gekennzeichnet, dass** die Zuordnung in Abhängigkeit von der Änderungsgeschwindigkeit und wenigstens einem weiteren Parameter erfolgt.

11. Verfahren nach einem der vorstehenden Ansprüche, **dadurch gekennzeichnet, dass** der Rauschparameter die absolute Intensität der Störsignale charakterisiert.

12. Verfahren nach einem der vorstehenden Ansprüche, **dadurch gekennzeichnet, dass** aus mehreren Messsignalen jeweils ein Messwert berechnet wird und aus mehreren Messwerten jeweils ein Wert des Rauschparameters berechnet wird.

13. Verfahren nach Anspruch 12, **dadurch gekennzeichnet, dass** die Messwerte durch Mittelwertbildung aus mehreren Messsignalen berechnet werden.

14. Verfahren nach einem der vorstehenden Ansprüche, **dadurch gekennzeichnet, dass** die Änderungsgeschwindigkeit des Rauschparameters an einer geglätteten Serie von Werten des Rauschparameters bestimmt wird.

## Claims

1. Method for detecting a malfunction of a sensor for measuring an analyte concentration in vivo, wherein
a series of measuring signals is produced by means of the sensor, and
a value of a noise parameter is successively determined from the measuring signals, the noise parameter indicating how severely the measurement is impaired by interference signals, **characterized in that** values of the noise parameter that are being determined successively are used to determine how quickly the noise parameter changes and the rate of change of the noise parameter is analyzed in order to detect a malfunction of the sensor.

2. Method according to claim 1, **characterized in that** one value of a noise parameter each is determined for a predetermined time interval.

3. Method according to any of the preceding claims, **characterized in that** a recursive filter is used to continually determine a value of a noise parameter from the measuring signals.

4. Method according to any of the preceding claims, **characterized in that** the rate of change of the noise parameter is analyzed by comparing it to a threshold value.

5. Method according to claim 4, **characterized in that** the threshold value is changed during sensor operation as a function of a measuring result.

6. Method according to claim 5, **characterized in that** the sensor comprises a working electrode, a counter-electrode, and a reference electrode, whereby the measuring result is based on a measurement of the electrical potential of the counter-electrode.

7. Method according to any one of the claims 4 to 6, **characterized in that**, after detecting that a value of the rate of change exceeds the threshold value, at least one subsequent value of the rate of change is analyzed to check if the exceeding of the threshold value is significant.

8. Method according to any of the preceding claims, **characterized in that** a warning signal is generated as a consequence of a malfunction being detected.

9. Method according to any of the preceding claims, **characterized in that** a malfunction that has been determined by analysis of the rate of change is assigned to one of at least two classes.

10. Method according to claim 9, **characterized in that** the assignment is made as a function of the rate of change and of at least one further parameter.

11. Method according to any of the preceding claims, **characterized in that** the noise parameter characterizes the absolute intensity of the interference signals.

12. Method according to any of the preceding claims, **characterized in that** multiple measuring signals are used to calculate one measuring value each and multiple measuring values are used to calculate one value of the noise parameter each.

13. Method according to claim 12, **characterized in that** the measuring values are calculated by calculating the mean of multiple measuring signals.

14. Method according to any of the preceding claims, **characterized in that** the rate of change of the noise parameter is determined on a smoothened series of values of the noise parameter.

## Revendications

1. Procédé de détection d'un dysfonctionnement d'un capteur permettant la mesure in vivo de la concentration d'un analyte, dans lequel on génère une série de signaux de mesure avec le capteur, et
on détermine en continu à partir des signaux de mesure une valeur d'un paramètre de bruit, qui indique à quel point la mesure est perturbée par des signaux parasites, **caractérisé en ce que** l'on détermine à partir de valeurs du paramètre de bruit déterminées en continu à quelle vitesse le paramètre de bruit varie, et on évalue la vitesse de variation du paramètre de bruit pour détecter un dysfonctionnement du capteur.

2. Procédé selon la revendication 1, **caractérisé en ce que** l'on détermine une valeur du paramètre de bruit chaque fois pour un intervalle de temps prédéfini.

3. Procédé selon l'une des revendications précédentes, **caractérisé en ce que** l'on détermine en continu une valeur du paramètre de bruit à partir des signaux de mesure avec un filtre récursif.

4. Procédé selon l'une des revendications précédentes, **caractérisé en ce que** l'on évalue la vitesse de variation du paramètre de bruit par comparaison avec une valeur seuil.

5. Procédé selon la revendication 4, **caractérisé en ce que** l'on modifie la valeur seuil pendant le fonctionnement du capteur en fonction d'un résultat de mesure.

6. Procédé selon la revendication 5, **caractérisé en ce que** le capteur comprend une électrode de travail, une contre-électrode et une électrode de référence, le résultat de mesure reposant sur une mesure du potentiel électrique de la contre-électrode.

7. Procédé selon l'une des revendications 4 à 6, **caractérisé en ce qu'**après constatation qu'une valeur de la vitesse de variation dépasse la valeur seuil, on vérifie par évaluation d'au moins une valeur suivante de la vitesse de variation si le dépassement est significatif.

8. Procédé selon l'une des revendications précédentes, **caractérisé en ce que** l'on produit comme conséquence de la détection d'un dysfonctionnement un signal d'avertissement.

9. Procédé selon l'une des revendications précédentes, **caractérisé en ce que** l'on associe un dysfonctionnement déterminé par évaluation de la vitesse de variation à une d'au moins deux classes.

10. Procédé selon la revendication 9, **caractérisé en ce que** l'association est faite en fonction de la vitesse de variation et d'au moins un autre paramètre.

11. Procédé selon l'une des revendications précédentes, **caractérisé en ce que** le paramètre de bruit caractérise l'intensité absolue des signaux parasites.

12. Procédé selon l'une des revendications précédentes, **caractérisé en ce que** l'on calcule à partir de plusieurs signaux de mesure chaque fois une valeur de mesure et on calcule à partir de plusieurs valeurs de mesure chaque fois une valeur du paramètre de bruit.

13. Procédé selon la revendication 12, **caractérisé en ce que** l'on calcule les valeurs de mesure en établissant la moyenne de plusieurs signaux de mesure.

14. Procédé selon l'une des revendications précédentes, **caractérisé en ce que** l'on détermine la vitesse de variation du paramètre de bruit sur une série de valeurs lissée du paramètre de bruit.
